# EUROPEAN PATENT SPECIFICATION

(11) **EP 2 556 378 B1**
(45) Date of publication and mention of the grant of the patent: **18.05.2016**
(21) Application number: 11776061.1
(22) Date of filing: 26.07.2011
(51) Int. Cl.: G01N 33/68, G01N 33/52, G01N 33/58

(54) **REAL-TIME MONITORING OF DEPLETION OF HIGH-ABUNDANCE BLOOD PROTEINS OR RECOVERY OF LOW-ABUNDANCE BLOOD PROTEINS BY UV SPECTROMETRY**
ECHTZEITÜBERWACHUNG DER ENTLADUNG VON BLUTPROTEINEN MIT HOHER DICHTE ODER DER WIEDERHERSTELLUNG VON BLUTPROTEINEN MIT GERINGER DICHTE DURCH UV-SPEKTROMETRIE
SURVEILLANCE EN TEMPS RÉEL DE LA DÉPLÉTION DES PROTÉINES DU SANG DE HAUTE ABONDANCE OU DE RÉCUPÉRATION DES PROTÉINES DU SANG DE FAIBLE ABONDANCE PAR SPECTROMÉTRIE UV

(30) Priority: 26.07.2010 KR 20100072142
(43) Date of publication of application: 13.02.2013
(73) Proprietor: SNU R&DB Foundation, Seoul 151-742 (KR)
(72) Inventor:
(74) Representative: Schiweck, Weinzierl & Koch
(86) International application number: PCT/KR2011/005500
(87) International publication number: WO 2012/015219

(56) References cited:
- WO-A2-01/81924
- JP-A- 2006 325 528
- KR-A- 20040 006 404
- KR-A- 20100 046 168
- Jiaxin Dong ET AL: "Development of Two Multiplex Immunoassays and Preliminary Application in Determining Protein Depletion for Seppro IgY14/SuperMix Depletion Columns", , 16 June 2010 (2010-06-16), XP055064739, Retrieved from the Internet: URL:http://www.sigmaaldrich.com/etc/medial ib/docs/Sigma-Aldrich/General_Information/ 1/seppro_article.Par.0001.File.tmp/seppro_ article.pdf [retrieved on 2013-05-30]
- VERONIKA POLASKOVA ET AL: "High-abundance protein depletion: Comparison of methods for human plasma biomarker discovery", ELECTROPHORESIS, vol. 31, no. 3, 1 January 2010 (2010-01-01), pages 471-482, XP055064716, ISSN: 0173-0835, DOI: 10.1002/elps.200900286
- ZOLOTARJOVA NINA ET AL: "Differences among techniques for high-abundant protein depletion", PROTEOMICS, WILEY - VCH VERLAG, WEINHEIM, DE, vol. 5, no. 13, 1 August 2005 (2005-08-01) , pages 3304-3313, XP008134114, ISSN: 1615-9853, DOI: 10.1002/PMIC.200402021 [retrieved on 2005-07-29]
- KIM KYUNGGON ET AL: "Online monitoring of immunoaffinity-based depletion of high-abundance blood proteins by UV spectrophotometry using enhanced green fluorescence protein and FITC-labeled human serum albumin", PROTEOME SCIENCE, BIOMED CENTRAL, LONDON, GB, vol. 8, no. 1, 1 December 2010 (2010-12-01), page 62, XP021088607, ISSN: 1477-5956, DOI: 10.1186/1477-5956-8-62
- DUKJIN KANG ET AL.: 'Hollow Fiber Flow Field-Flow Fractionation of Proteins Using a Microbore Channel.' ANAL. CHEM. vol. 77, no. 13, 2005, pages 4207 - 4212
- TAO LIU ET AL.: 'Evaluation of Multiprotein Immunoaffinity Subtraction for Plasma Proteomics and Candidate Biomarker Discovery Using Mass Spectrometry.' MOLECULAR & CELLULAR PROTEOMICS. vol. 5, no. 11, 2006, pages 2167 - 2174

## Description

### [Technical Field]

The present invention relates to a method for monitoring the depletion of high-abundance proteins and recovery of low-abundance proteins from blood in real time, comprising
(a) labeling high-abundance proteins of a blood specimen with a fluorescent or UV marker which is selected from the group consisting of FITC (Fluorescein isothiocyanate), TRITC (tetramethyl-rhodamine isothiocyanate), Cy3, Cy5 and Rhodamine and spiking low-abundance proteins of a blood specimen with a fluorescent or UV marker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP); and
(b) passing blood samples containing the high-abundance proteins to be depleted which are labelled with said fluorescent or UV marker which is selected from the group consisting of FITC (Fluorescein isothiocyanate), TRITC (tetramethyl-rhodamine isothiocyanate), Cy3, Cy5 and Rhodamine and low-abundance proteins which are spiked with said fluorescent or UVmarker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP) through a removal column.

### [Background Art]

Blood, a representative body fluid, serves as a barometer for indicating the state of health of the body because the changes in the components of the blood are sensitive to the onset and progress of diseases. The plasma of the blood is the blood without the blood cells and circulates all over the body, and is highly likely to contain protein hydrolysates and various early biomarkers from each tissue. Hence, research has attempted to ascertain early markers of diseases by analyzing the plasma proteins. Among these, about 30 proteins are present at high levels, making it difficult to detect biomarkers by protein analysis. Over 50,000 kinds of proteins are expected to be contained in blood over a highly dynamic concentration range (1-10¹²). It is therefore very difficult to qualitatively and quantitatively analyze biomarker candidates of low to moderate levels using only LC (liquid chromatography)-MS/MS, which has a detection limit of as low as 10⁴⁻⁶. One of the most important steps for finding disease biomarkers in the blood is the minimization of the complexity of samples. For this, columns (e.g., a Multiple Affinity Removal System, or MARS) or fractionation based on various properties of proteins (pI values, molecular weight, hydrophilicity, etc.) are used to deplete high-abundance proteins such as albumin, immunoglobulins, etc. Particularly, one of the most efficient strategies for identifying biomarker candidates present at a very low level is obtaining as large a protein fraction as possible using Multiple Affinity Removal System (MARS) to minimize sample complexity, followed by carrying out qualitative or quantitative analysis with a mass spectrometry. Also, PF2DTM was found to be very effective in the fractionation and analysis of proteins as well as membrane proteins and peptides.

To detect a protein biomarker out of the plasma proteins, it is necessary to deplete abundant proteins of high molecular weights. Conventionally, albumin and immunoglobulin G (IgG) are depleted using a column which of resin is conjugated with corresponding antibodies. However, kits for depleting albumin and IgG cannot guarantee the level of protein recovery. As for the antibody columns used in the depletion of six different high-abundance proteins, they could mis-capture at least 16 high-abundance proteins, and this makes the analysis of low-abundance plasma proteins difficult.

To overcome the problems stemming from the use of antibodies to separate high-abundance proteins, an ultrafiltration method using a cut-off membrane was developed, but has not yet been applied to protein analysis because its buffer system cannot promise the level of protein recovery and because it causes the degradation of proteins.

Radhakrishna S. et al. suggested a purification method for low molecular weight plasma proteins in which a cut-off membrane is used to deplete MAHP (major high molecule weight proteins) from blood plasma in combination with 25mM ammonium bicarbonate and 20% acetonitrile buffer. However, this method also suffers from the disadvantage of recovering degraded proteins.

Various methods have been developed that deplete high-abundance proteins from the blood. Most of these are based on the immune affinity of antibodies for high-abundance proteins. For instance, in spite of several reports on the problem about incomplete depletion of target high abundant proteins and unintended depletion of low abundant proteins, multiple affinity removal system (MARS, Agient, CA, USA) columns are frequently used to prepare blood samples. In depletion of high abundant protein of blood, the reproducibility of removal processes and the recovery ability of low-level proteins are important factors in the elimination of high-abundance proteins. However, there are no property-controlling processes that can be conveniently used for the monitoring of these parameters. Several techniques such as BCA (bicinchoninic acid) analysis, ELISA (enzyme-linked immunosorbent assay), etc. have been used to determine the extent of depletion, but these post-depletion experiments require significant cost and time. In this context, Jiaxin Dong et al: "Development of Two Multiplex Immunoassays and Preliminary Application in Determining Protein Depletion for Seppro IgY14/SuperMix Depletion Columns", 16 June 2010 disclose the Depletion of Highly Abundant Protein (HAP) and Moderately Abundant Protein (MAP) from plasma or serum samples can be achieved through two Sigma Seppro Tandem IgY14/ SuperMix columns. The first column contains IgY antibodies against 14 Highly Abundant Proteins (HAP)in order to immobilize these HAPs. The moderately abundant proteins are present in the flow-through of the first column. The second column is a IgY-SuperMix Column that contains IgY antibodies against the Moderately Abundant Proteins (MAP) in order to these MAPs.

In addition, despite its utility, the depletion of high-abundance proteins bears with it an intrinsic problem, called the sponge effect, which must be considered in the experiments. The sponge effect refers to the concomitant depletion of some of the low-level proteins upon the separation of high-abundance proteins from blood by columns, and thus acts as a factor that has a negative influence on the separation yield of low-level blood proteins. In addition, the depletion of the high-abundance proteins must be monitored over the entire period that the removal columns are used for. No suitable techniques have yet been provided for measuring the recovery rate of low-level proteins after depletion.

### [Disclosure]

### [Technical Problem]

Culminating in the present invention, intensive and thorough research into the real time monitoring of the specific depletion of high-abundance proteins and/or recovery of low-abundance proteins from blood, was conducted by the present inventors. As a result, the present invention was created after confirming that as an easy and quick examination method for checking recovery and reproducibility of a MARS column performance, the method using EGFP (Enhanced green fluorescence protein) and FITC (fluorescein isothiocyanate) as an indicator of low-abundance proteins and high-abundance proteins, respectively, can monitor the amount of depletion for albumin, a high-abundance protein.

### [Technical Solution]

It is therefore an object of the present invention to provide a method for monitoring depletion of high-abundance proteins and recovery of low-abundance proteins from blood in real time, comprising:
(a) labeling high-abundance proteins of a blood specimen with a fluorescent or UV marker which is selected from the group consisting of FITC (Fluorescein isothiocyanate), TRITC (tetramethyl-rhodamine isothiocyanate), Cy3, Cy5 and Rhodamine and spiking low-abundance proteins of a blood specimen with a fluorescent or UV marker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP); and
(b) passing blood samples containing the high-abundance proteins to be depleted which are labelled with said fluorescent or UV marker which is selected from the group consisting of FITC (Fluorescein isothiocyanate), TRITC (tetramethyl-rhodamine isothiocyanate), Cy3, Cy5 and Rhodamine and low-abundance proteins which are spiked with said fluorescent or UVmarker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP through a removal column.

### [Advantageous Effects]

The monitoring method of the present invention can improve the efficiency with which high-abundance proteins are depleted from a blood specimen and can contribute to the biomarker research field by reducing experimental errors, such as the sponge effect, thus allowing the selective separation of low-abundance proteins from blood specimens. In addition, the monitoring of FITC-labeled human albumin at UV488nm makes it feasible to trace the depletion and recovery of high-abundance proteins conducted with HPLC-based depletal systems including MARS.

### [Description of Drawings]

The above and other objects, features and other advantages of the present invention will be more clearly understood from the following detailed description taken in conjunction with the accompanying drawings, in which:

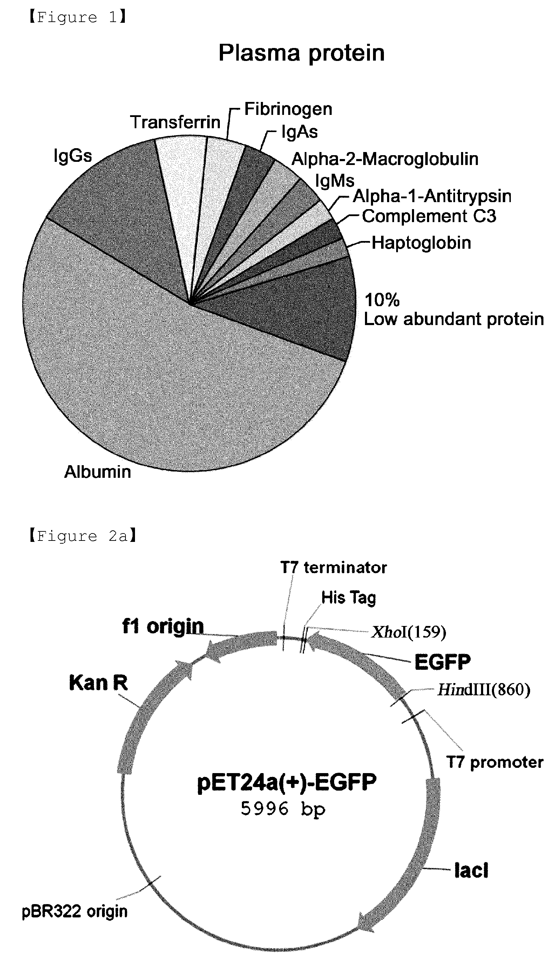

FIG. 1 is a graph showing the distribution of high-abundance proteins within plasma;

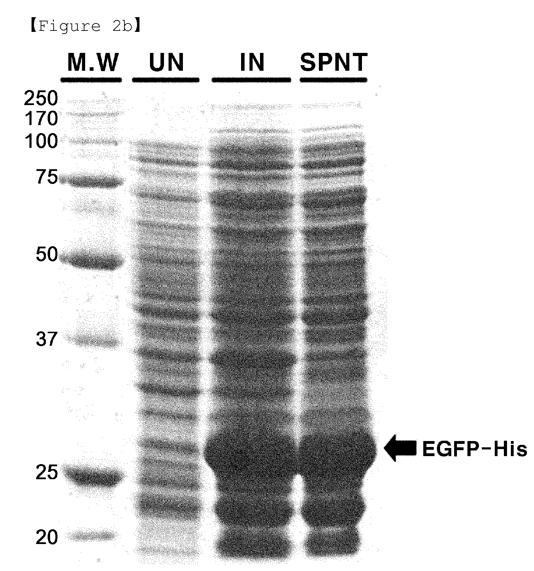

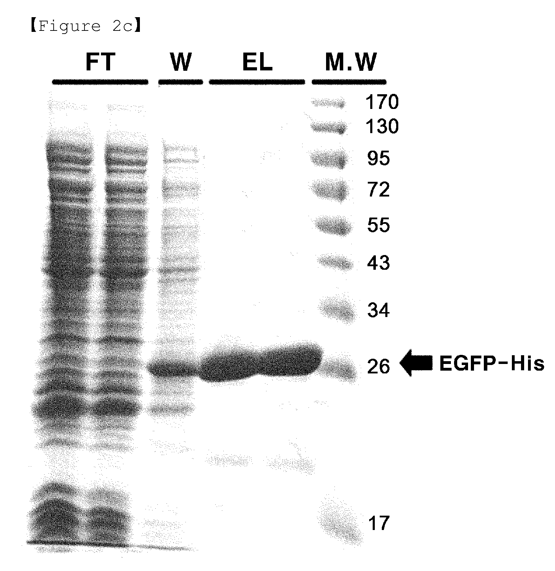

FIG. 2 shows the cloning, overexpression, and purification of EGFP. (a) A map of pET24a (+)-EGFP-His was drawn with vector NTI software (version 6.0, InforMax). The EGFP gene is indicated by the gray arrow. (b) pET24a (+)-EGFP-His was transformed into BL21 RIL codon-plus (DE3) (Novagen) and overexpressed by IPTG induction. A molecular weight marker (MW), an uninduced total cell lysate (UN), an induced total cell lysate (IN), and the supernatant of an induced cell lysate (SPNT) were loaded on a 12% SDS-PAGE gel and visualized with Coomassie Brilliant Blue R250. (c) Purification was performed by Ni-NTA, and the flow-through fraction (FT), wash fraction (W), and eluted fraction (EL) were run on a 12% SDS-PAGE gel. The EGFP-His band is indicated by a black arrow;

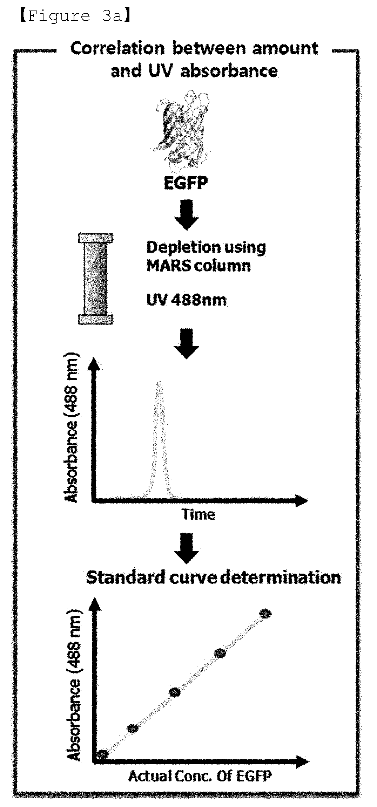

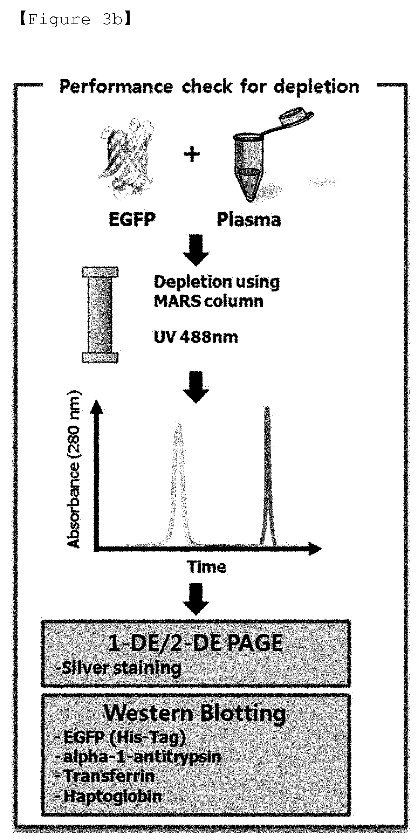

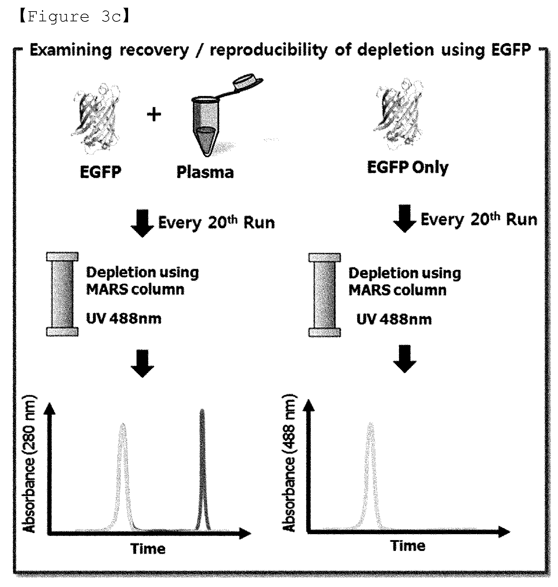

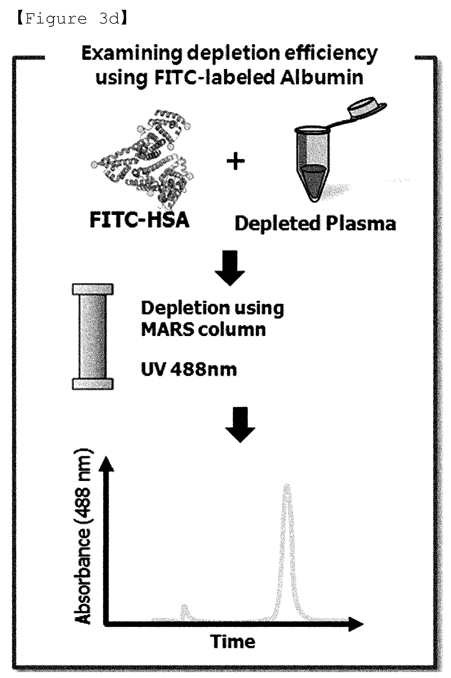

FIG. 3 is a schematic view of all experiments. (a) The correlation between the quantity of EGFP and UV absorbance, (b) the recovery of EGFP-spiked plasma and performance check for depletion, (c) The recovery and reproducibility of depletion upon the use of EGFP as an indicator of low-abundance proteins, and (d) Depletions upon the use of FITC-labeled HSA as a high-abundance protein indicator;

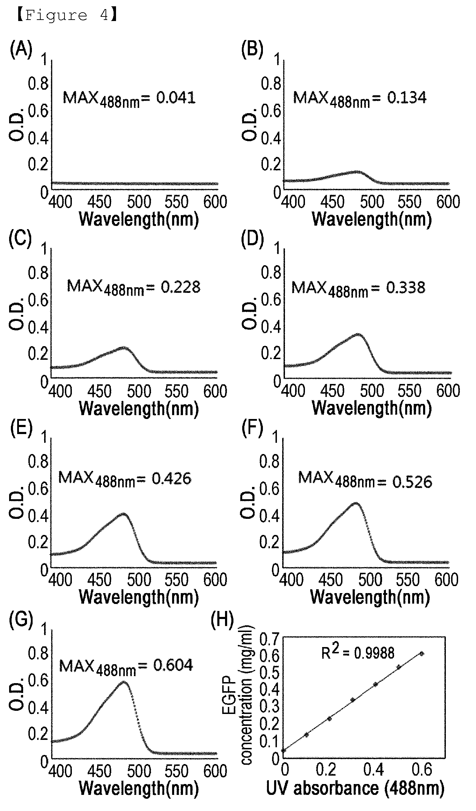

FIG. 4 is a view showing the linear correlation of UV absorbance at 488 nm versus EGFP concentration. EGFP was diluted from 0.1 mg/ml to 0.6 mg/ml in MARS buffer A. The UV absorbance of 200 *µ*ℓ of diluted samples was measured in a wavelength range of 400 nm to 600 nm, on an ELISA reader. (A) Blank, MARS buffer A, (B) 0.1 mg/ml (20 *µg),* (C) 0.2 mg/ml (40 *µ*g), (D) 0.3 mg/ml (60 *µ*g), (E) 0.4 mg/ml (80 *µ*g), (F) 0.5 mg/ml (100 *µ*g) , and (G) 0.6 mg/ml (120 *µ*g). (H) Linear correlation of UV absorbance versus EGFP concentration was calculated by linear regression analysis algorithm. The correlation of coefficient (R²) is 0.9988;

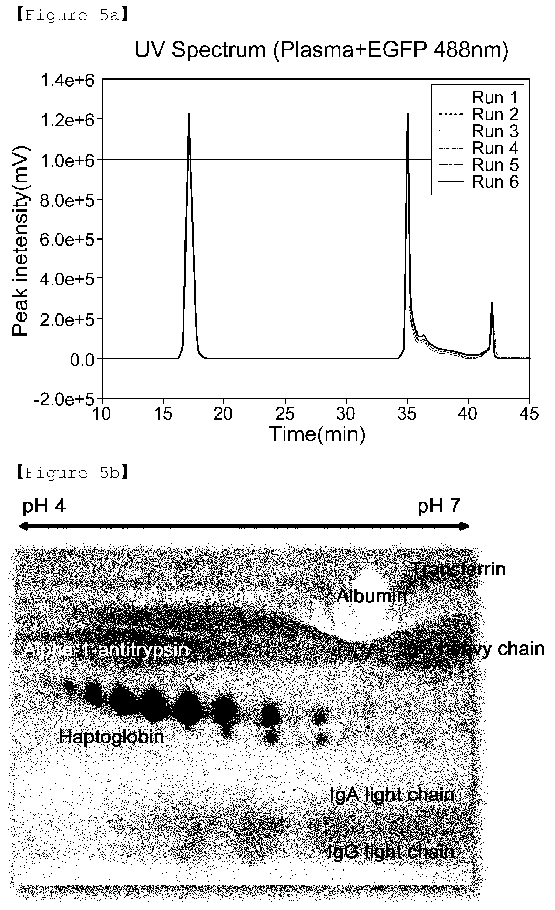

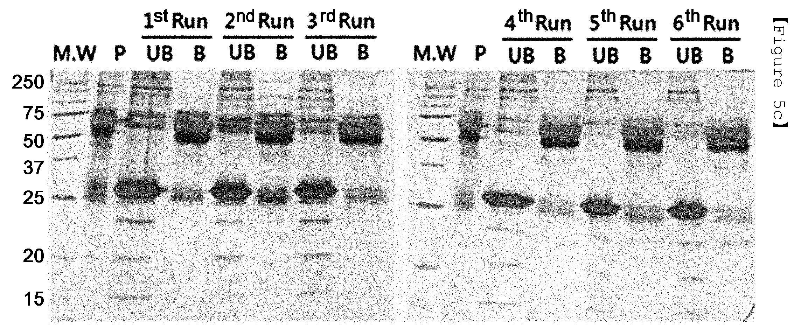

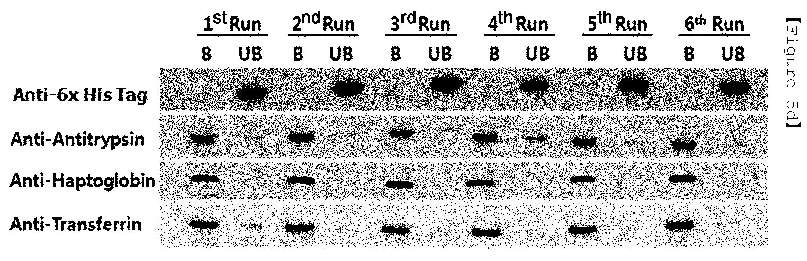

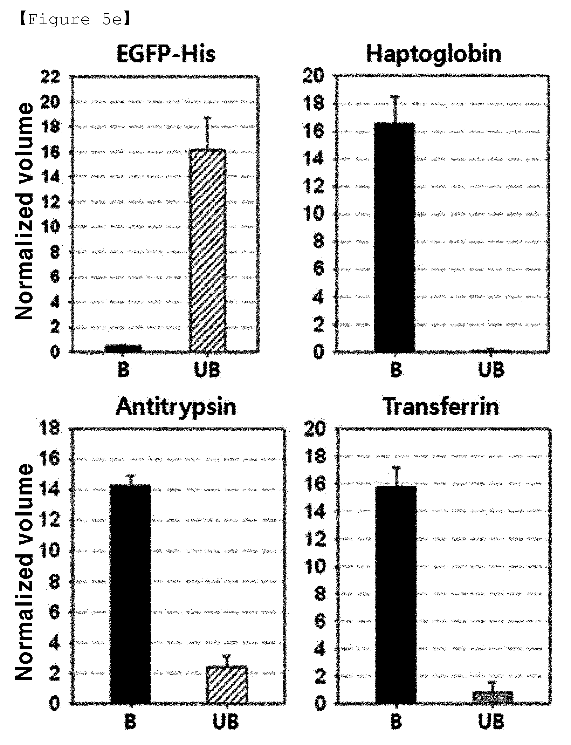

FIG. 5 shows the performance of EGFP-spiked plasma in depletion experiments. (a) Overlay of chromatograms from 6 consecutive MARS depletion runs using EGFP-spikedplasma. (b) 30 *µ*g of the bound fraction (high-abundance proteins) was subjected to 2-DE PAGE. Six high-abundance proteins on a silver-stained gel were visualized. (c) Equal amounts (5 *µ*g) of plasma (P), an unbound fraction (UB, low-abundance proteins), and a bound fraction (B, high-abundance proteins) were run on an 8-12% SDS-PAGE gel and visualized by silver staining. (d) The unbound fraction (UB) and the bound fraction (B) were analyzed by Western blot to detect target proteins. Anti-6xHis tag was used to detect His-tagged EGFP, and antibodies for antitrypsin, haptoglobin, and transferrin were used for the bound and unbound fractions from the 6 consecutive depletion runs. (e) Each protein in the Western blots was expressed as band intensity and shown on a bar graph with standard errors, using a gel analysis program;

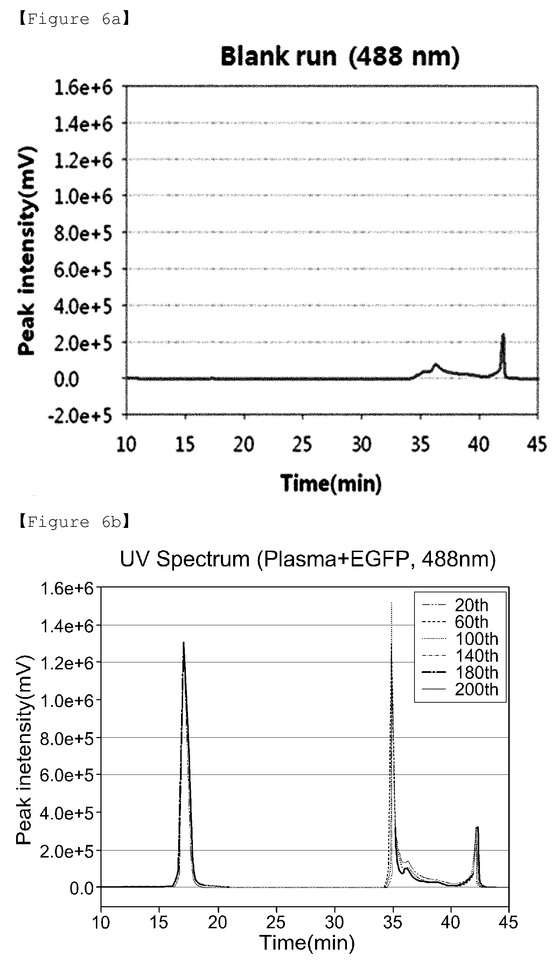

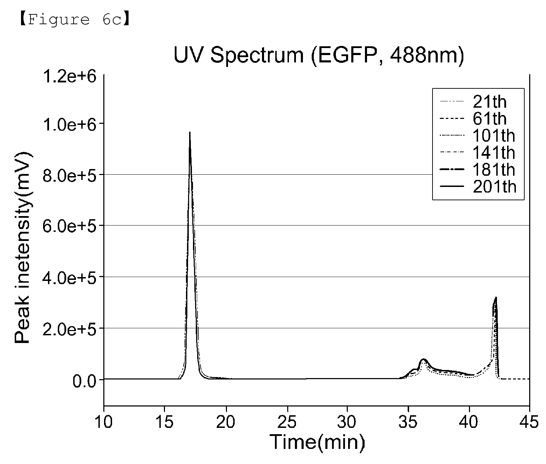

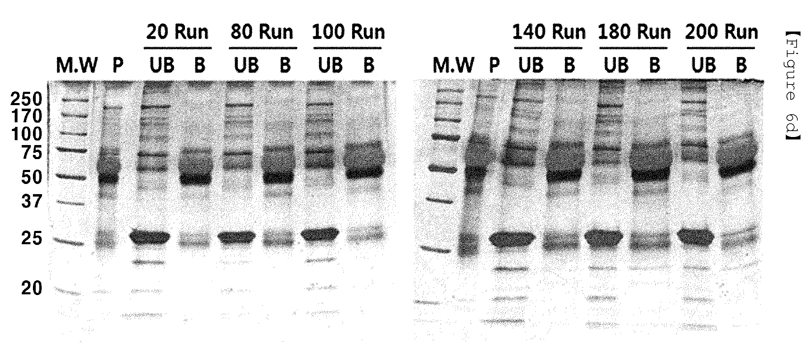

FIG. 6 shows recovery upon the use of EGFP as a low-abundance protein indicator. (a) 100 microliters of MARS buffer A was injected into the MARS column as the blank run. This spectrum at UV488 nm generated 2 peaks at 37 min and 43 min from the buffer components. Thus, these 2 peaks were ignored in subsequent experiments. (b) An overlay of chromatograms from the 20^{th}, 60^{th}, 100^{th}, 140^{th}, 180^{th}, and 200^{th} MARS column runs using EGFP-spiked plasma. (c) An overlay of chromatograms from the 21^{st}, 61^{st},101^{st}, 141^{st}, 181^{st}, and 201^{st} MARS column runs using EGFP-only. (d) Equal amounts (5 *µ*g) of plasma (P), an unbound fraction (UB), and a bound fraction (B) were loaded onto an 8-12% SDS-PAGE gel and visualized by silver staining; and

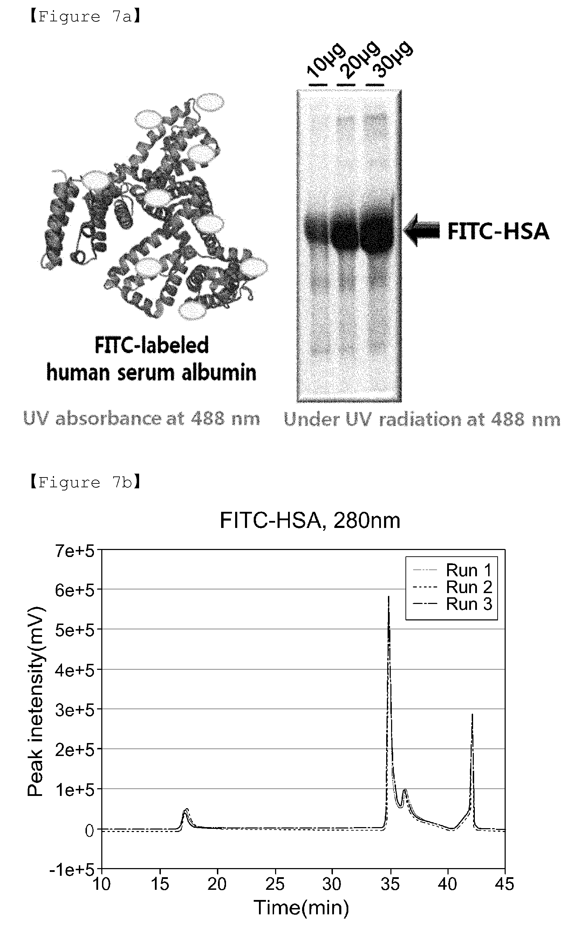

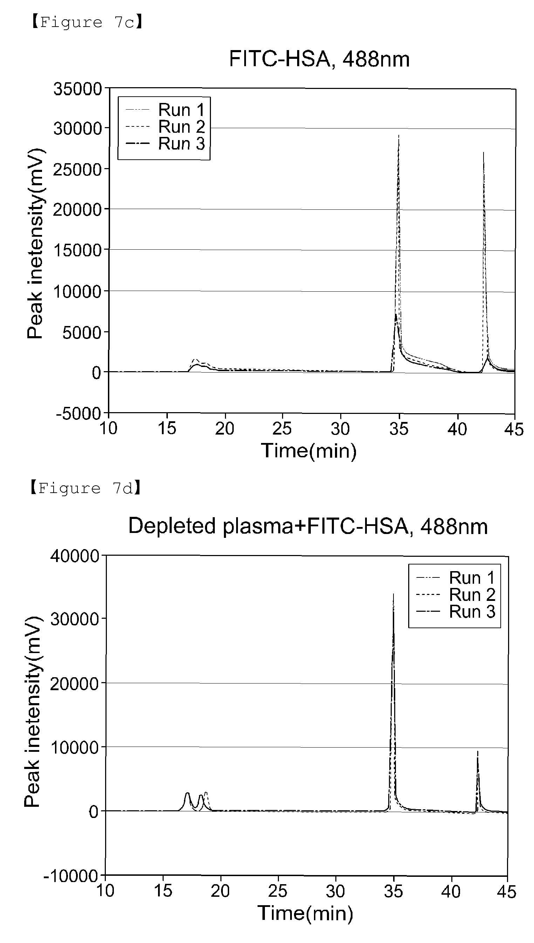

FIG. 7 shows depletion efficiency upon the use of FITC-labeled albumin as a high-abundance protein indicator. (a) Structural model of FITC-labeled HSA visualized under UV_{488 nm} illumination on SDS-PAGE and the amount of FITC-labeled HSA. (b) Chromatograms at UV₂₈₀ₙₘ in the depletion runs using FITC-labeled HSA as a high-abundance protein indicator. (c) Chromatograms at UV₄₈₈ₙₘ in depletion runs using FITC-labeled HSA as the high abundance protein indicator. (d) Chromatograms at UV₄₈₈ₙₘ in depletion using FITC-labeled HSA-spiked depleted plasma. At first, the most abundant six proteins (HSA, immunoglobulin G, alpha-1-antitrypsin, immunoglobulin A, transferrin, and haptoglobin) in plasma were depleted and FITC-labeled HSA which of amount is equal to endogenous HSA was spiked. Then, the depletion yield of high abundance protein which can be representative by FITC-labeled HSA was monitored during MARS column performance.

### [Best Mode]

In accordance with an aspect thereof, the present invention provides a method for monitoring the depletion of high-abundance proteins and/or recovery of low-abundance proteins from blood in real time, comprising:
(a) labeling high-abundance proteins of a blood specimen with a fluorescent or UV marker which is selected from the group consisting of FITC (Fluorescein isothiocyanate), TRITC (tetramethyl-rhodamine isothiocyanate), Cy3, Cy5 and Rhodamine and spiking low-abundance proteins of a blood specimen with a fluorescent or UV marker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP); and
(b) passing blood samples containing the high-abundance proteins to be depleted which are labelled with said fluorescent or UV marker which is selected from the group consisting of FITC (Fluorescein isothiocyanate), TRITC (tetramethyl-rhodamine isothiocyanate), Cy3, Cy5 and Rhodamine and low-abundance proteins which are spiked with said fluorescent or UVmarker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP) through a removal column.

As used herein, the term "blood specimen" refers to blood, blood plasma itself or the blood plasma from which the blood cells have been removed. Blood plasma is composed of water, proteins, lipids, carbohydrates, and other inorganic ions. Out of the plasma proteins, albumin and globulin may be discriminated from each other due to a difference in their solubility in ammonium sulfate. Like albumin, globulin encompasses a heterogeneous group of proteins. Serum is plasma without fibrinogen. To be distinguished from those in plasma, albumin or globulin in serum may be called serum albumin or serum globulin. Albumin accounts for approximately 55% of the volume of all plasma proteins. Albumin serves as a protein source and mainly functions to regulate the colloidal osmotic pressure of blood. Globulin constitutes approximately 38% of the volume of all plasma proteins. α-globulin contains lipoproteins and glycoproteins and is used to transport vitamins and hormones. β-globulin functions to carry prothrombin, plasma thromboplastin, iron and copper. γ-globulin is composed mainly of immunoglobulin. Fibrinogen is responsible for blood coagulation. Over 90% of the plasma proteins are high-abundance proteins while about 10% may be used as biomarkers.

The term "biomarker, " as used herein, refers to a substance which indicates disease states and genetic characteristics of individual patients, particularly to an indicator of the biological change caused by a post-natal genetic change, detectable from the molecular information based on single molecules derived from DNA, RNA, metabolites, or protein fragments, or on patterns of the molecules. The term "high-abundance protein, " as used herein, refers to a protein present in a large amount in plasma. Collectively, high-abundance proteins amount to over 90% of the mass of the plasma proteins. Examples of the high-abundance protein include, but are not limited to, albumin, immunoglobulin G (IgG), alpha-1-antitrypsin, immunoglobulin A (IgA), transferrin, haptoglobin, fibrinogen, α-2-macroglobulin, immunoglobulin M (Ig M) and complement C3. Examples and mass ratios of the plasma proteins are depicted in FIG. 1. Further, examples of the high-abundance protein include, but are not limited to, apolipoprotein A-I, apolipoprotein A-II, apolipoprotein B, α-1-Acid Glycoprotein, ceruloplasmin, complement C4, complement C1q, immunoglobulin D (IgD), prealbumin and plasminogen (Electrophoresis 2010, 31, 471-482). In a preferred embodiment, albumin, a representative high-abundance protein, is labeled with a fluorescent protein to monitor the depletion thereof.

The term "fluorescent or UV marker," as used herein, refers to a substance or a site thereof that is detectable by fluorescence or UV within a detectable range. These markers include proteins that can be detected by fluorescence or UV within or compounds that show fluorescence to UV within a specific wavelength range. Examples of proteins used as fluorescence or UV markers include a green fluorescent protein (GFP), a modified green fluorescent protein (mGFP), an enhanced green fluorescent protein (EGFP), a red fluorescent protein (RFP), a modified red fluorescent protein (mRFP), an enhanced red fluorescent protein (ERFP), a blue fluorescent protein (BFP), an enhanced blue fluorescent protein (EBFP), a yellow fluorescent protein (YFP), an enhanced yellow fluorescent protein (EYFP), a cyan fluorescent protein (CFP) and an enhanced cyan fluorescent protein (ECFP), but are not limited thereto. FITC (Fluorescein isothiocyanate), TRITC (tetramethyl-rhodamineisothiocyanate),Cy3 (Cyanine3), Cy5 (Cyanine 5) or rhodamine may be also used as a fluorescence or UV indicator. Preferably, high-abundance proteins or low-abundance proteins may be labeled with FITC or EGFP. The method may further comprise spiking blood with a fluorescent or UV marker different from the fluorescent or UV marker applied to the high-abundance or low-abundance proteins. The fluorescent or UV marker may be selected from those able to response to detectable wavelength bands used in accordance with the design of experiments.

As used herein, "low-abundance protein" refers to a protein component in blood specimen, exclusive of high-abundance proteins. And it can be useful as a biomarker of a specific disease. In spite of its utility, using a low-abundance protein as a biomarker is subject to extreme limitations because they are difficult to detect. In the present invention, the depletion of high-abundance proteins and recovery or low-abundance proteins with a removal column is monitored in real time so that the optimal conditions for the recovery of a biomarker can be established.

The term "removal column," as used herein, refers to a column which allows the absorption and removal of undesired proteins from a blood specimen by immunoaffinity similar to the specificity of antigen-antibody response so as to separate a protein of interest. Column conditions, such as fillers, buffers and other separation environments, under which this general principle of recovering proteins of interest is carried out, may vary depending on various factors including the kinds, properties and molecular weights of the proteins. Preferably, a MARS column equipped with the multiple affinity removal system may be used to separate high-abundance proteins from a blood specimen.

Blood plasma (blood from which the blood cells have been removed) is treated with two fluorescent markers with 488nm wavelengths and then allowed to pass through a column capable of selectively absorbing fluorescent marker-labeled proteins, to deplete high-abundance proteins from the plasma. Thereafter, the depletion efficiency of the high-abundance proteins is monitored in real time using the fluorescent markers attached to the proteins (FIGS. 3a to 3d). In greater detail, high-abundance proteins in a plasma specimen are labeled with the UV-detectable fluorescent marker FITC and low-abundance proteins are spikedwithEGFP, which wavelength is 488nm, after which the fluorescent marker-labeled plasma components are allowed to pass through a MARS column to selectively and specifically deplete the high-abundance proteins of albumin, immunoglobulin G, alpha-1-antitrypsin, immunoglobulin A, transferrin and haptoglobin. The FITC attached to the high-abundance proteins is analyzed with a UV wavelength of 488 nm. As a result, the FITC-labeled, high-abundance proteins are observed to have been effectively depleted by the MARS column (FIGS. 7a to 7d). In addition, the recovery of low-abundance proteins from blood was analyzed (FIGS. 6a to 6d and Tables 1 to 3) . An EGFP standard curve is established as a control and used to compare the recovery of the EGFP-labeled, low-abundance proteins (FIG. 4). The depleted, high-abundance proteins are identified by subjecting them to one-dimensional electrophoresis and silver staining. As a result, they are identified as albumin, immunoglobulin G, alpha-1-antitrypsin, immunoglobulin A, transferrin and haptoglobin (FIGS. 5a to 5e). Of the six proteins, alpha-1-antitrypsin, haptoglobin and transferrin are analyzed for depletion efficiency by Western blotting. It is found that haptoglobin is completely depleted by the column (FIGS. 5d and 5e).

The step of labeling high-abundance proteins in a blood specimen with a fluorescent or UV marker may comprise expressing a vector carrying a gene coding for a fluorescence protein and a gene coding for the high-abundance. That is, a fusion protein may be expressed.

The term "vector, " as used he re in, refers to a nucleic acid molecule used as a vehicle to transfer a genetic material, which is a spliced foreign nucleic acidmolecule. One type of useful vector is an episome, a nucleic acid molecule with which a gene can be cloned outside the chromosome. Useful vectors are those that can autonomously carry and/or express genes linked thereto. A vector which allows the detectable expression of a gene operably linked thereto is called an "expression vector". Generally, an expression vector useful in recombinant DNA technology is in the form of a "plasmid." A plasmid is a DNAmolecule that is separate from, and can replicate, independently of, the chromosomal DNA. In many cases, they are double-stranded loops. They can be easily constructed by those skilled in the art.

As used herein, the term "operably linked" refers to the linkage of nucleic acid fragments in such a functional relationship that when expressed, each of them can operate without functional problems, whereas, when one neucleic acid fragment is connected to the other, its function and expression may be affected by the other. In addition, vectors can include random regulation sequence of transcription initiation and transcription termination to regulate transcription. Two genetic elements can be operably linked to each other using gene recombination technology. Site-specific DNA cleavage and linkage can be achieved using enzymes known in the art.

The step of labeling high-abundance proteins in a blood specimen with a fluorescent or UV marker may further comprise binding a fluorescent or UV marker to a peptide specific for a high-abundance protein, and conjugating the high-abundance protein with the fluorescence or UV marker-bound peptide.

As used herein the term "peptide" refers to a polymer of two or more amino acids. In general, a short polymer of few amino acids is called a peptide and a long polymer of many amino acids is called a protein. The amino acids of peptides and proteins are linked by peptide or amide bonds, which are formed by dehydration and condensation between carboxyl groups and amino groups.

To deplete high-abundance proteins from a blood specimen, a peptide capable of binding specifically to the high-abundance proteins is used. A non-limiting, illustrative example is amyloid αβ 40 (Biochemical and Biophysical Research Communications, 365, 2007, 714-718). Amyloid αβ 40 can bind specifically and effectively to the high-abundance protein albumin in plasma. Like this, specific peptides which match the high-abundance proteins by 1 to 1 ratio make it possible to monitor protein depletion and recovery with greater efficiency because they introduce the marker more accurately into the proteins.

The step of labeling high-abundance proteins in a blood specimen with a fluorescent or UV marker may further comprise binding a fluorescent or UV marker to an aptamer specific binding affinity for a high-abundance protein, and conjugating the high-abundance protein with the fluorescence or UV marker-bound aptamer.

As used herein the term "aptamer" refers to a nucleic acid ligand which can adopt a specific three-dimensional conformation suitable for binding to high-abundance or low-abundance proteins to form a complex therewith. Typically, the aptamer may be a short nucleic acid molecule 15 ~ 50 nucleotide in length which is folded into a predetermined secondary or tertiary structure, e.g., a stem-loop structure. Preferably, aptamers bind at a kd less than 10⁻⁶, 10⁻⁸, 10⁻¹⁰, or 10⁻¹² to target molecules, high-abundance or low-abundance proteins. The specificity of the aptamer for target molecules may be very high. Further, the aptamer may consist of a number of ribonucleotide units, deoxyribonucleotide units or a mixture of the two nucleotide units. Also, the aptamer may further comprise one or more modified base, sugar or phosphate backbone units.

High-abundance proteins in a blood specimen are depleted using a MARS column in which a column filler shows immunoaffinity for the high-abundance proteins. To monitor the depletion efficiency thereof, the proteins are labeled with FITC that is detectable at a UV wavelength of 488 nm. In a preferred example of the monitoring method, two or more independent fluorescence or UV markers are employed in a separate manner, or simultaneously and in sequence.

High-abundance proteins proteins are labeled with different respective fluorescent markers and are detected at different wavelengths so as to monitor the depletion and recovery of each protein. In detail, high-abundance proteins to be depleted and low-abundance proteins to be isolated are labeled with FITC and spiked with EGFP, respectively. Therefore, it is confirmed that the depletion and separation of those proteins can be monitored at specific UV wavelengths which correspond to their indicators.

The monitoring method may be performed with the simultaneous treatment of a blood specimen with two or more fluorescence-or UV-detectable different fluorescence proteins which can be simultaneously monitored at detectable wavelength. Specially, wherein two or more fluorescence-or UV-detectable proteins can be mixed in blood simultaneously, and monitored at corresponding detection wavelength ranges at the same time, wherein the fluorescence- or UV-detectable proteins are complex of high-abundance proteins, low-abundance proteins, or high-abundance proteins and low-abundance proteins, respectively.

### [Mode for Invention]

A better understanding of the present invention may be obtained through the following examples.

### EXAMPLE 1: Preparation of Plasma Specimen

To reduce sampling bias due to the difference among individuals, 100 *µ*ℓ plasma samples were taken from each of ten healthy humans (five men and five women), followed by centrifugation at 15,000 rpm at 4°C for 10 min to remove debris.

The centrifuged sample was divided into aliquots of 50 *µ*ℓ and stored at -80°C until use. The aliquots were thawed at 4°C for use in depletion experiments. Human plasma samples were provided from the Seoul National University Hospital, Korea. All of the subject individuals consented to participate in this research, and experiments were carried out according to the protocol approved by the Institutional Review Board of the Seoul National University Hospital, Korea.

### EXAMPLE 2: Expression of EGFP (Enhanced Green Fluorescence Protein)

EGFP was cloned, expressed and purified (FIGS. 2a to 2c). Information on a nucleotide sequence of EGFP was obtained from the internet site of Lab Life (http://www.lablife.org). Overall EGFP-encoding sequence was obtained by performing PCR on pEGFP N1 (Clonetech, CA, USA) in the presence of the sense primer 5'-GACAAGCTTATATGGTGAGCA-3'(SEQ ID NO: 1) and the antisense primer 5'-GCCGGGATCACTCTCGAGCAC-3'(SEQ ID NO: 2). In the primers, underlined italic letters denote HindIII and XhoI restriction enzyme sites, respectively, while the bold letters are additional bases accounting for the generation of an open reading frame. After digestion with the restriction enzymes, the PCR products were excised from the electrophoresis gel and ligated to a pET 24a (+) vector (Novagen, WI, USA).

The resulting recombinant vector pET24a(+)-EGFP-His was transformed into BL21 RIL codon-plus cells ((DE3) (Novagen)). The transformant BL21-CodonPlus (DE3)-RIL (5 mL) anchoring the EGFP DNA structure therein was grown overnight, inoculated (5 mL) into 200 mL of LB broth containing kanamycin (30 *µ*g/mL) and incubated overnight at 37°C. After the cells had been grown to an optical density of 1.0 at 600nm, 0.5 mM IPTG (isopropyl β d-thiogalactopyranoside) was added at 21°C to induce the cells to express the protein of interest.

After induction overnight, the cell culture was centrifuged at 5000 g for 10 min. The cell pellet thus obtained was suspended in a ice-cold 20 mM TrisHCl (pH 7.8) containing 200mM NaCl, 1mM beta-mercaptoethanol(βME), and a protease inhibitor cocktail (Roche, Swiss). The cell suspension was disrupted by ultrasonication. The cell supernatant was collected by centrifugation at 15,000 g and 4°C for 20 min. EGFP was purified using Ni-NTA. EGFP was quantified using UV₄₈₈ₙₘ as in BCA assay.

### EXAMPLE 3: Determination of EGFP Standard Curve Using UV488nm

To draw out a standard curve for EGFP, EGFP was dissolved and diluted sequentially from 0 to 0.6 mg/mL (by 0.1 mg/mL, 7 points) in MARS buffer A (Agilent, CA, USA). To 96-well plates (SPL, Korea) was added 200 *µ*ℓ of each of the diluted samples, followed by measuring absorbance at a wavelength range of from 400 nm to 600 nm on an ELISA reader (PowerWave XSBio-Tek, VT, USA). A standard curve was drawn from the already known amounts of EGFP and from the observed absorbance values.

### EXAMPLE 4: Labeling of Human Serum Albumin with FITC

Human serum albumin (HSA, Cat No. A1543-10G) andFITC (fluorescein isothiocyanate, Cat. No. F7250-1G) were purchased from Sigma Aldrich (St. Louis, MO, USA). A solution of 100 *µ*g of HSA in 50 mL of 100 mM sodium carbonate was mixed at a molar ratio of 1:1 with a solution of 0.585 g of FITC in 1 mL of DMSO (dimethyl sulfoxide) (each 1.5 µM), followed by incubation overnight at 4°C.

FITC-labeled HSA was concentrated with a 5-kDa cut-off centricon filter (Millipore, MA, USA) and dialyzed against PBS overnight using a Slide-A-Lyzer dialysis cassette (Pierce, Rockford, IL, USA). Re-concentration through a 5-kDa cut-off centricon filter depleted unbound FITC. FITC-labeled HSA was concentrated into a volume of 500 *µ*ℓ and purified by gel filtration on a Superdex S-200 column using an AKTA FPLC system (Amersham Biosciences, Uppsala, Sweden) to enhance the purity.

### EXAMPLE 5 : Depletion of High-abundance proteins from Human Plasma by MARS Column Chromatography

To deplete plasma using the MARS column, plasma was diluted 5-fold in MARS buffer A and filtered on a 0.22-µm Ultrafree-MC Durapore centrifugal filter. To deplete the most abundant six proteins (human serum albumin, immunoglobulin G, alpha-1-antitrypsin, immunoglobulin A, transferrin, haptoglobin), EGFP-spiked plasma, and EGFP-only samples were applied to a 4.6 mm x 10 mm-size MARS column (Product No. 5185-5985, Agilent) on an LC-10AT HPLC system (Shimadzu, Kyoto, Japan).

58 *µ*ℓ of 5-fold diluted plasma and 42 *µ*ℓ of EGFP (final concentration of 0.3 mg/ml) or 58 *µ*ℓ of MARS buffer A and 42 *µ*ℓ of EGFP (final concentration of 0.3 mg/ml) were injected into the MARS column. The total LC run time of 45 min comprised the following sequence: 100% MARS buffer A at a flow rate of 0.7 ml/min for an initial 15 min, sample injection, a 15-min wash, 100% MARS buffer B at a flow rate of 1.0 ml/min for 5 min, and 100% MARS buffer A at a flow rate of 0.7 ml/min for 10 min: The UV detector was set to 280 nm or 488 nm, and the eluted fractions were collected every 500 *µ*ℓ on fraction collector.

To determine whether FITC-labeled HSA was separable on a MARS column, 600 *µg* of FITC-labeled HSA was run on a MARS column while the UV detector was set to 488 nm. The depletion process was performed 3 consecutive times with FITC-labeled HSA. Furthermore, to verify the depletion of FITC-labeled HSA from plasma, the depleted plasma (6 high-abundance proteins were depleted, including HSA) was mixed with 600 *µg* of FITC-labeled HSA to simulate HSA-containing undepleted plasma, which was then separated 3 consecutive times on a MARS column with the UV detector set to 488 nm.

### EXAMPLE 6 : Quantification of Bound or Unbound Plasma Protein from the Removal Column

During plasma depletion, the volumes of the unbound fraction and the bound fraction were 2.5 ml and 5.0 ml, respectively. Each fraction was pooled and concentrated to 1.0 mL on a Nanosep 3K centrifugal filter (Pall Corporation, NY, USA). Afterwards, 200 *µ*ℓ of the concentrates was transferred to 96-well plates (SPL, Korea) to read UV absorbance at 488 nm on an ELISA reader.

### EXAMPLE7 : 2-Dimensional Gel Electrophoresis and Silver Staining

IEF (isoelectric focusing) was carried out using an IPGphore system (Amersham Biosciences, Uppsala,Sweden). Bound fractions were pooled and buffer-exchanged with 5 mL of rehydration buffer (7 M urea, 2 M thiourea, 2% CHAPS, 60 mM DTT, and 0.5% (v/v) pharmalyte (pH 3-10)). Thirty micrograms of high-abundance proteins in the pooled, bound fraction was subjected to IEF using immobilized pH gradient (IPG) strips (7 cm, pH 4-7, linear gradient; Amersham Biosciences, Sweden), as described in Wang Y, Cheung YH, Yang Z, et al. Proteomic approach to study the cytotoxicity of dioscin (saponin). Proteomics. 2006;6:2422-2432. The protein spots were visualized by silver staining and scanned on a UMAX PowerLook 2100 XL (UMAX Technologies, TX).

### EXAMPLE 8 : Western Blot Analysis

For Western blot analysis, the concentrated bound fraction in MARS buffer B was buffer-exchanged with MARS buffer A on a Nanosep 3K centrifugal filter because MARS buffer B reacts with a BCA solution to generate color. After BCA analysis, 5 *µ*g of samples was separated on 12% SDS-PAGE gels and transferred to PVDF membranes for 1 hr at 4°C in the presence of 100 V (GE Healthcare, NJ, USA).

Thereafter, the membranes were blocked with 5% (w/v) BSA, 0.1% Tween 20 in TBS buffer for 2 hours at room temperature and incubated overnight at 4°C with primary antibodies. The primary antibodies included mouse anti-human transferrin (1:1000; AbFrontier), mouse anti-human alpha-1-antitrypsin (1:1000; AbFrontier), mouse anti-human haptoglobin(1: 1000;AbFrontier), and rabbit anti-Hisprobe (2:1000; Santa Cruz Biotechnology, CA, USA).

After being washed in 0.5% Tween 20 in TBS, the membranes were incubated with peroxidase-conjugated secondary antibodies (Santa Cruz) at 1:5000 for 1 hr at room temperature. Signals were detected using ECL Plus Western Blotting Detection Reagents (GE Healthcare) on an LAS-4000 Image analyzer (Fujifilm, Tokyo, Japan). The signals were analyzed quantitatively using image analysis software (2D Phoretix expression v2004, Newcastle, UK). All results were expressed as means and analyzed statistically by Student's t-test.

### EXPERIMENTAL EXAMPLE 1 : Correlation between EGFP Level and UV Absorbance (FIGS. 3a and 4)

For use in depletion experiments, EGFP was cloned, overexpressed and purified to obtain proper quantity of batch (FIGS. 2a to 2c). Thereafter, EGFP concentrations were measured to examine the linearity of EGFP between 0.1 mg/mL and 0.6 mg/mL (0.1-mg/ml intervals) versus UV₄₈₈ₙₘ absorbance. UV absorbance was read between 400 nm and 600 nm (FIGS. 4A to 4G). EGFP concentrations showed an excellent linear relationship versus UV absorbance at 488 nm, as proved by R²=0.9988 (FIG. 4H).

### EXPERIMENTAL EXAMPLE 2 : Performance of EGFP-spiked plasma during depletion (FIGS. 3b and 5)

To examine the reproducibility of EGFP as a flow-through indicator in plasma on the MARS column, depletion experiments were performed six consecutive times, wherein 58 *µ*ℓ of diluted plasma plus 42 *µ*ℓ of EGFP was applied onto the MARS column. The chromatograms for the six runs were reproducible with regard to retention time and UV intensity for EGFP-spiked plasma (FIG. 5a).

The bound and unbound fractions in the six depletion experiments were also examined by SDS-PAGE (FIG. 5c). The number and shape of the SDS-PAGE bands were identical to those of a previous report. In addition, bands of the bound fractions containing the six high-abundance proteins were analyzed using MALDI-TOF/TOF (data not shown). The bound fractions from the six depletion experiments was subjected to 2-DE; the 2-DE patterns were observed to be consistent with a previous study (FIG. 5b). Consequently, the MARS column was observed to show reproducibility along with the EGFP-spiked plasma.

Next, we validated the efficacy with which the MARS column depletes by analyzing the bound and unbound fractions of EGFP-spiked plasma for the presence of representative high-abundance proteins, such as α-1-antitrypsin, haptoglobin, and transferrin, and low-abundance proteins, such as EGFP-containing His-tag, by Western blot (FIG. 5d) .

Three (α-1-antitrypsin, haptoglobin, and transferrin) of the six high-abundance proteins were selected for Western blot analysis because the silver-stained bands of albumin and immunoglobulin by SDS-PAGE were abundant enough to be visualized.

In order to be detected by Western blot, anti-His was used to a probe for EGFP, because it contained a His-tag for purification purposes. Also, EGFP was not necessary for depletion by the MARS column, and thus should be detected only in the unbound fractions.

EGFP was detected only in the unbound fraction. Therefore, it did not undergo the albumin sponge effect or carry over to the bound fraction. Clearly, α-1-antitrypsin and transferrin were not depleted completely in the unbound fractions while haptoglobin was nearly depleted from the unbound fraction (FIG. 5d). These results verify a previous study in which incomplete depletion was observed in the unbound fraction. The Western blot analysis results were expressed in bar graphs with standard error marks using Phoretix gel analysis software (FIG. 5e).

### EXPERIMENTAL EXAMPLE 3 : Reproducibility of Depletion Using EGFP-Spiked Plasma and EGFP Alone (FIGS. 3c and 6)

The present inventors determined the reproducibility of depletion with EGFP-only or EGFP-spiked plasma within the recommended use of MARS columns (200 runs) . Injection samples were prepared in the repeat experiments in the same manner as in the previous section. EGFP-spiked plasma was injected into the MARS column every 20^{th} run in the range from the 20^{th} to 200^{th} run (FIG. 6b) and EGFP-only sample was injected every 20th run in the range from the 21st to 201st (FIG. 6c).

The flow-through and high-abundance protein fractions were identified by UV chromatograms and SDS-PAGE analysis of the six depletion experiments (FIG. 6d). In these UV chromatograms, the peaks of the unbound fractions (low-abundance proteins) and the bound fractions (high-abundance proteins) appeared reproducibly at 16 min and 35 min, respectively, in all batches. Two peaks at retention times of 36 min and 42 min also developed in all runs, but they did not harbor proteins, because they were detected in the blank run with an identical peak height and retention time (FIG. 6a). All 6 runs confirmed that depletion by the MARS column was reproducible for up to 201 cycles.

### EXPERIMENTAL EXAMPLE 4 : Recovery Rate of Depletion Using EGFP as Low-Abundance Protein Indicator (FIG. 3c and Tables 1-3)

The effect of the eluted EGFP on the recovery of EGFP after the depletion in the MARS column run was examined. The amount of EGFP that was spiked into the plasma sample was adjusted to 0.3 mg/mL wherein the volume of the EGFP-containing unbound fraction was reduced to 1 mL (42 *µ*ℓ EGFP in 1 mL buffer, that is, 0.3 mg. UV absorbance was measured using 200 *µ*ℓ of each concentrated sample to calculate the depletion recovery. At an EGFP concentration of 0.3 mg/ml, the 200 *µ*ℓ sample has an UV₄₈₈ₙₘ of 0.3888 (FIGS. 4D and 4H).

This concentration (0.3 mg/ml) is in the middle of the standard curve in the concentration range of from 0.1 to 0.6 mg/ml.

The depletion by the MARS column using EGFP-spiked plasma and EGFP-only was summarized every 20th run in Tables 1 and 2, below. The coefficient of variation for the peak area of the low-abundance proteins was 8.5% for each run, which was higher for the high-abundance protein (23.2%, bound fraction) (Table 1). The high variation in the bound fraction peak was caused by a fluctuation in the 4th run. In Table 1, the recovery of EGFP in EGFP-spiked plasma using EGFP as a low-abundance protein indicator was summarized.

**[Table 1]**

| **Run** | **O.D_{488 nm}^{a)}** | **EGP conc. (mg/ml)^{b)}** | **Recovery (%)^{c)}** | **Retention Time (min)** | **Peak Area** | **O.D_{488 nm}^{d)}** | **Retention Time (min)** | **Peak Area** |
|---|---|---|---|---|---|---|---|---|
| | **Unbound fraction** | | | | | **Bound fraction** | | |
| **20** | 0.318 | 0.288 | 96.108 | 17.204 | 41318386 | 0.042 | 35.119 | 21663431 |
| **40** | 0.310 | 0.280 | 93.291 | 17.198 | 41864592 | 0.042 | 35.132 | 20405735 |
| **60** | 0.316 | 0.286 | 95.404 | 17.130 | 46612910 | 0.043 | 35.003 | 27522817 |
| **80** | 0.314 | 0.284 | 94.700 | 17.234 | 48029530 | 0.043 | 35.087 | 40458786 |
| **100** | 0.313 | 0.283 | 94.348 | 17.116 | 47410534 | 0.042 | 34.888 | 29504690 |
| **120** | 0.302 | 0.271 | 90.474 | 17.117 | 42958986 | 0.042 | 34.900 | 24795107 |
| **140** | 0.309 | 0.279 | 92.939 | 17.157 | 44253260 | 0.042 | 34.918 | 23585379 |
| **160** | 0.312 | 0.282 | 93.995 | 17.143 | 37695839 | 0.043 | 34.889 | 21280293 |
| **180** | 0.314 | 0.284 | 94.700 | 17.119 | 38865664 | 0.042 | 34.906 | 21914998 |
| **200** | 0.317 | 0.287 | 95.756 | 17.166 | 39476099 | 0.042 | 34.916 | 25579936 |
| **Average** | **0.313** | **0.283** | **94.172** | **17.158** | **42848580.000** | **0.042** | **34.976** | **25671117.200** |
| **S.D.** | **0.0047** | **0.0049** | **1.6456** | **0.042** | **3665255.437** | **0.0005** | **0.100** | **5954454.913** |

| | | | | | | | | |
|---|---|---|---|---|---|---|---|---|
| a) The unbound fractions were concentrated to a volume of 1 ml, at which the UV absorbance was measured at 488 nm. b) EGFP concentration was calculated using the standard curve, as shown in Figure 4H. c) EGFP recovery EGFP. concentration/0.3 × 100. d) The bound fractions were concentrated to a volume of 1 ml, at which the UV absorbance was measured at 488 nm. | | | | | | | | |

The coefficients of variation for UV absorbance, EGFP concentration, recovery and retention time were lower than 2%, demonstrating that each run with EGFP is highly reproducible. The average recovery of EGFP was 94.2% for EGFP-spiked plasma (Table 1) and 93.8% for EGFP-only (Table 2). Moreover, EGFP was detected only in the unbound fraction whereas the bound fraction contained no traces of EGFP (FIGS. 5c, 5d and 6d). These results indicate that EGFP can serve as an indicator of low-abundance proteins in plasma. In Table 2, the recovery of EGFP from EGFP-only solution using EGFP as a low-abundance protein indicator is summarized.

**[Table 2]**

| **Run** | **O.D_{488 nm} ^{a)}** | **EGFP conc. (mg/ml)^{b)}** | **Recovery (%)^{d}** | **Retention Time (min)** | **Peak Area** | **O.D_{488 nm}^{d)}** | **Retention Time (min)** | **Peak Area** |
|---|---|---|---|---|---|---|---|---|
| | **Unbound fraction** | | | | | **Bound fraction** | | |
| **21** | 0.323 | 0.294 | 97.869 | 17.176 | 27072418 | 0.042 | 36.265 | 12098039 |
| **41** | 0.296 | 0.265 | 88.361 | 17.158 | 25303785 | 0.042 | 36.316 | 12924057 |
| **61** | 0.314 | 0.284 | 94.700 | 17.128 | 26898491 | 0.043 | 36.408 | 7177513 |
| **81** | 0, 316 | 0.286 | 95.404 | 17.229 | 27372996 | 0.043 | 36.665 | 5887288 |
| **101** | 0.313 | 0.283 | 94.348 | 17.137 | 27712834 | 0.042 | 36.387 | 5351547 |
| **121** | 0.309 | 0.279 | 92.939 | 17.142 | 25688223 | 0.042 | 36.220 | 8400685 |
| **141** | 0.307 | 0.277 | 92.235 | 17.154 | 27835164 | 0.043 | 36.146 | 9345686 |
| **161** | 0.310 | 0.280 | 93.291 | 17.152 | 23774802 | 0.043 | 36.106 | 5814103 |
| **181** | 0.313 | 0.283 | 94.348 | 17.124 | 27535453 | 0.042 | 36.149 | 5474879 |
| **201** | 0.314 | 0.284 | 94.700 | 17.139 | 27848972 | 0.041 | 36.216 | 5325185 |
| **Average** | **0.312** | **0.281** | **93.819** | **17.154** | **26704313.800** | **0.042** | **36.288** | **7779898.200** |
| **S.D.** | **0.0070** | **0.0074** | **2.4582** | **0.030** | **1353730.325** | **0.0007** | **0.167** | **2847817.672** |

| | | | | | | | | |
|---|---|---|---|---|---|---|---|---|
| a) The unbound fractions were concentrated to a volume of 1 ml, at which the UV absorbance was measured at 488 nm. b) EGFP concentration was calculated using the standard curve, as shown in FIG. 4H. c) EGFP recovery (%) = EGFP concentration/0.3 × 100. d) The bound fractions were concentrated to a volume of 1 ml, at which the UV absorbance was measured at 488 nm. | | | | | | | | |

The data support that EGFP is useful for monitoring the recovery of low-abundance proteins during MARS column chromatography. As described in Experimental Example 3, the recovery of EGFP was calculated in the six consecutive depletions and is summarized in Table 3, below. Table 3 shows the recovery of EGFP in EGFP-spiked plasma during six consecutive depletions.

**[Table 3]**

| **Run** | **O.D₄₈₈ₙₘ^{a)}** | **EGFP conc. (mg/ml)^{b)}** | **Recovery (%)^{c)}** | **Retention Time (minute)** | **Peak Area** |
|---|---|---|---|---|---|
| **1** | 0.323 | 0.294 | 97.869 | 17.153 | 42853087 |
| **2** | 0.313 | 0.283 | 94.348 | 17.135 | 42241736 |
| **3** | 0.323 | 0.294 | 97.869 | 17.133 | 42173830 |
| **4** | 0.301 | 0.270 | 90.122 | 17.129 | 41879936 |
| **5** | 0.319 | 0.289 | 96.461 | 17.135 | 41974861 |
| **6** | 0.316 | 0.286 | 95.404 | 17.168 | 38653456 |
| **Average** | **0.316** | **0.286** | **95.345** | **17.142 416** | **629484.333** |
| **S.D.** | **0.0083** | **0.0087** | **2.9077** | **0.015 14** | **497138.839** |

| | | | | | |
|---|---|---|---|---|---|
| a) The unbound fractions were concentrated to a volume of 1 ml, at which the UV absorbance was measured at 488 nm. b) EGFP concentration was calculated using the standard curve, as shown in FIG. 4H. c) EGFP recovery (%) = EGFP concentration/0.3 × 100. d) The data was obtained from the experiment of FIG. 5. | | | | | |

### EXPERIMENTAL EXAMPLE 5 : Monitoring the Efficiency of Depletion Using FITC-Labeled Albumin as an Indicator of High-Abundance Proteins (FIG. 3d)

To examine the ability of the MARS column to deplete high-abundance proteins, FITC-labeled albumin was used as an indicator of high-abundance proteins. FITC is detected optimally at UV_{488 nm} with which albumin and the other five high-abundance proteins can be monitors. AfterpurifiedHSAwas labeledwithFITC, FITC-labeledHSAwas separated using SDS-PAGE to assess the purity and fluorescence thereof (FIG. 7a). Then, 600 *µ*g of FITC-labeled HSA was applied in triplicate onto the MARS column while UV monitoring was performed at 280 nm and 488 nm.

Three repeat depletion experiments were performed using FITC-HSA, which yielded nearly identical UV absorption spectra at 280 nm and 488 nm (FIGS. 7b and 7c). FITC-labeled HSA, bound to the MARS column, was eluted at a retention time of 35 min. Two peaks at 36 min and 42 min did not contain proteins, because they were also detected in the blank run.

To determine whether FITC-labeled HSA is useful as a high abundance protein indicator in plasma, six high-abundance proteins, including human serum albumin (HSA), were depleted from plasma using the MARS column, and the depleted plasma was mixed with 600 *µg* of FITC-labeled HSA as an indicator. Presumably, this amount corresponds to the HSA content in 16 *µ*ℓ of plasma, and the volume is the same as that applied to the MARS column in this study. Moreover, the amount of protein was assumed to be 70 *µ*g per 1 *µ*ℓ plasma and the percentage of HSA to be about 50%.

The depleted plasma containing FITC-labeled HSA was applied to the MARS column, and UV monitoring at 488 nm was performed three times (FIG. 7d). The elution patterns of three repeat runs were similar overall, whereas the small peak between 18 min and 19 min was delayed slightly in the second run compared to the first and third (FIG. 7d). This was considered as a minor variation. The peak at the retention time of 17 min in both experiments (FITC-labeled HSA, and FITC-labeled HSA contained in depleted plasma, FIGS. 7c and 7D) reflected small amounts of unbound FITC-HSA. In consideration of the fact that there were no peaks in the unbound fraction in the blank run (data not shown), it was speculated that this phenomenon was caused by epitopes blocking in HSA by FITC labeling.

When FITC is conjugated to HSA, the ability of the MARS column resin to capture might decrease. HSA labeled with UV-detectable fluorescent proteins, such as RFP (red) and CFP (blue), instead of FITC, might increase the efficiency of monitoring the depletion of high-abundance proteins.
## Claims

1. A method for monitoring depletion yield of high-abundance proteins and recovery yield of low-abundance proteins from blood in real time, comprising:
(a) labeling high-abundance proteins of a blood specimen with a fluorescent or UV marker which is selected from the group consisting of FITC (Fluorescein isothiocyanate), TRITC (tetramethyl-rhodamine isothiocyanate), Cy3, Cy5 and Rhodamine and spiking low-abundance proteins of a blood specimen with a fluorescent or UV marker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP); and
(b) passing blood samples containing the high-abundance proteins to be depleted which are labelled with said fluorescent or UV marker which is selected from the group consisting of FITC (Fluorescein isothiocyanate), TRITC (tetramethyl-rhodamine isothiocyanate), Cy3, Cy5 and Rhodamine and low-abundance proteins which are spiked with said fluorescent or UV marker which is selected from the group consisting of green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), cyan fluorescent protein (CFP) and enhanced cyan fluorescent protein (ECFP) through a removal column.

2. The method of claim 1, wherein the high-abundance protein is selected from the group consisting of albumin, immunoglobulin G (IgG), alpha-1-antitrypsin, immunoglobulin A, transferrin, haptoglobin, fibrinogen, α-2-macroglobulin, immunoglobulin M (Ig M) and complement C3, apolipoprotein A-I, apolipoprotein A-II, apolipoprotein B, α-1-acid glycoprotein, ceruloplasmin, complement C4, complement C1q, immunoglobulin D (IgD), prealbumin, plasminogen and a combination thereof.

3. The method of claim 1, wherein the labeling step of step (a) is carried out by tagging a peptide specific for the high-abundance proteins with the fluorescent or UV marker and binding this marker-tagged peptide to the high-abundance proteins.

4. The method of claim 1, wherein the labeling step of step (a) is carried out by tagging a aptamer specific for the high-abundance proteins with the fluorescent or UV marker and binding this marker-tagged aptamer to the high-abundance proteins.

5. The method of claim 3, wherein the high-abundance protein is albumin and the peptide specific for the high-abundance protein is amyloid αβ40.

6. The method of claim 1, wherein the protein is monitored independently at different detection wavelength bands.

7. The method of claim 1, wherein two or more fluorescence- or UV-detectable proteins can be mixed in blood simultaneously, and monitored at corresponding detection wavelength ranges at the same time, wherein the fluorescence- or UV-detectable proteins are complex of high-abundance proteins, low-abundance proteins, or high-abundance proteins and low-abundance proteins, respectively.

## Patentansprüche

1. Verfahren zur Beobachtung der Depletionsrate von häufig vorkommendenden Proteinen und der Wiederfindungsrate von in niedriger Menge vorkommenden Proteinen aus Blut in Echtzeit, das Folgendes umfasst:
(a) Markieren von häufig vorkommenden Proteinen einer Blutprobe mit einem Fluoreszenz- oder UV-Marker, der aus der Gruppe ausgewählt ist, die aus FITC (Fluoresceinisothiocyanat), TRITC (Tetramethylrhodaminisothiocyanat), Cy3, Cy5 und Rhodamin besteht, und Versetzen ("Spiking") von niedrigabundanten Proteinen einer Blutprobe mit einem Fluorezenz- oder UV-Marker, der aus der Gruppe ausgewählt ist, die aus Folgenden besteht: grün fluoreszierendem Protein (GFP), modifiziertem grün fluoreszierendem Protein (mGFP), verstärktem grün fluoreszierendem Protein (EGFP), rot fluoreszierendem Protein (RFP), modifiziertem rot fluoreszierendem Protein (mRFP), verstärktem rot fluoreszierendem Protein (ERFP), blau fluoreszierendem Protein (BFP), verstärktem blau fluoreszierendem Protein (EBFP), gelb fluoreszierendem Protein (YFP), verstärktem gelb fluoreszierendem Protein (EYFP), cyan fluoreszierendem Protein (CFP) und verstärktem cyan fluoreszierendem Protein (ECFP); und
(b) Durchleiten der Blutproben, die Folgendes enthalten: die zu depletierenden häufig vorkommenden Proteine, die mit dem Fluoreszenz- oder UV-Marker markiert sind, der aus der Gruppe ausgewählt ist, die aus FITC (Fluoresceinisothiocyanat), TRITC (Tetramethylrhodaminisothiocyanat), Cy3, Cy5 und Rhodamin besteht, und die in niedriger Menge vorkommenden Proteine, die mit dem Fluoreszenz- oder UV-Marker bespickt sind, der aus der Gruppe ausgewählt ist, die aus Folgenden besteht: grün fluoreszierendem Protein (GFP), modifiziertem grün fluoreszierendem Protein (mGFP), verstärktem grün fluoreszierendem Protein (EGFP), rot fluoreszierendem Protein (RFP), modifiziertem rot fluoreszierendem Protein (mRFP), verstärktem rot fluoreszierendem Protein (ERFP), blau fluoreszierendem Protein (BFP), verstärktem blau fluoreszierendem Protein (EBFP), gelb fluoreszierendem Protein (YFP), verstärktem gelb fluoreszierendem Protein (EYFP), cyan fluoreszierendem Protein (CFP) und verstärktem cyan fluoreszierendem Protein (ECFP), durch eine Entfernungssäule.

2. Verfahren nach Anspruch 1, wobei das häufig vorkommende Protein aus der Gruppe ausgewählt ist, die aus Folgenden besteht: Albumin, Immunglobulin G (IgG), alpha-1-Antitrypsin, Immunglobulin A, Transferrin, Haptoglobin, Fibrinogen, α-2-Makroglobulin, Immunglobulin M (IgM) und Komplement C3, Apolipoprotein A-I, Apolipoprotein A-II, Apolipoprotein B, α-1-Säure-Glycoprotein, Ceruloplasmin, Komplement C4, Komplement C1q, Immunglobulin D (IgD), Prealbumin, Plasminogen und einer Kombination davon.

3. Verfahren nach Anspruch 1, wobei der Markierungsschritt von Schritt (a) durch "Tagging" eines Peptids, das für die hochabundanten Proteine spezifisch ist, mit dem Fluoreszenz- oder UV-Marker und Binden dieses Markergekennzeichneten Peptids an die häufig vorkommenden Proteine durchgeführt wird.

4. Verfahren nach Anspruch 1, wobei der Markierungsschritt von Schritt (a) durch "Tagging" eines Aptamers, das für die häufig vorkommenden Proteine spezifisch ist, mit dem Fluoreszenz- oder UV-Marker und Binden dieses Markergekennzeichneten Aptamers an die häufig vorkommenden Proteine durchgeführt wird.

5. Verfahren nach Anspruch 3, wobei das häufig vorkommende Protein Albumin ist und das Peptid, das für das häufig vorkommende Protein spezifisch ist, Amyloid αβ40 ist.

6. Verfahren nach Anspruch 1, wobei das Protein unabhängig bei unterschiedlichen Wellenlängenbändern zur Detektion beobachtet wird.

7. Verfahren nach Anspruch 1, wobei zwei oder mehr Fluoreszenz- oder UV-detektierbare Proteine im Blut gleichzeitig vermischt sein können und bei entsprechenden Wellenlängenbereichen zur Detektion zeitgleich beobachtet werden können, wobei die Fluoreszenz- oder UVdetektierbaren Proteine Komplexe aus häufig vorkommenden Proteinen, in geringer Menge vorkommenden Proteinen bzw. häufig vorkommenden Proteinen und in geringer Menge vorkommenden Proteinen sind.

## Revendications

1. Procédé de surveillance du rendement de déplétion de protéines à abondance élevée et du rendement de récupération de protéines à faible abondance à partir de sang en temps réel, comprenant :
(a) marquer des protéines à abondance élevée d'un échantillon de sang avec un marqueur fluorescent ou UV qui est sélectionné parmi le groupe constitué du FITC (isothiocyanate de fluorescéine), TRITC (isothiocyanate de tétraméthyl-rhodamine), Cy3, Cy5 et rhodamine et ajouter à des protéines à faible abondance d'un échantillon de sang un marqueur fluorescent ou UV qui est sélectionné parmi le groupe constitué de la protéine fluorescente verte (GFP), la protéine fluorescente verte modifiée (mGFP), la protéine fluorescente verte amplifiée (EGFP), la protéine fluorescente rouge (RFP), la protéine fluorescente rouge modifiée (mRFP), la protéine fluorescente rouge amplifiée (ERFP), la protéine fluorescente bleue (BFP), la protéine fluorescente bleue amplifiée (EBFP), la protéine fluorescente jaune (YFP), la protéine fluorescente jaune amplifiée (EYFP), la protéine fluorescente cyan (CFP) et la protéine fluorescente cyan amplifiée (ECFP) ; et
(b) faire passer des échantillons de sang contenant les protéines à abondance élevée devant être déplétées qui sont marquées avec ledit marqueur fluorescent ou UV qui est sélectionné parmi le groupe constitué du FITC (isothiocyanate de fluorescéine), TRITC (isothiocyanate de tétraméthyl-rhodamine), Cy3, Cy5 et rhodamine et les protéines à faible abondance auxquelles est ajouté ledit marqueur fluorescent ou UV qui est sélectionné parmi le groupe constitué de la protéine fluorescente verte (GFP), la protéine fluorescente verte modifiée (mGFP), la protéine fluorescente verte amplifiée (EGFP), la protéine fluorescente rouge (RFP), la protéine fluorescente rouge modifiée (mRFP), la protéine fluorescente rouge amplifiée (ERFP), la protéine fluorescente bleue (BFP), la protéine fluorescente bleue amplifiée (EBFP), la protéine fluorescente jaune (YFP), la protéine fluorescente jaune amplifiée (EYFP), la protéine fluorescente cyan (CFP) et la protéine fluorescente cyan amplifiée (ECFP) à travers une colonne d'élimination.

2. Procédé selon la revendication 1, dans lequel la protéine à abondance élevée est sélectionnée parmi le groupe constitué de l'albumine, l'immunoglobuline G (IgG), l'alpha-1-antitrypsine, l'immunoglobuline A, la transferrine, l'haptoglobine, le fibrinogène, l'α-2-macroglobuline, l'immunoglobuline M (Ig M) et le complément C3, l'apolipoprotéine A-I, l'apolipoprotéine A-II, l'apolipoprotéine B, l'α-1-glycoprotéine acide, la céruloplasmine, le complément C4, le complément C1q, l'immunoglobuline D (IgD), la préalbumine, le plasminogène et une combinaison de ceux-ci.

3. Procédé selon la revendication 1, dans lequel l'étape de marquage de l'étape (a) est effectuée en marquant un peptide spécifique aux protéines à abondance élevée avec le marqueur fluorescent ou UV et fixant ce peptide marqué par un marqueur aux protéines à abondance élevée.

4. Procédé selon la revendication 1, dans lequel l'étape de marquage de l'étape (a) est effectuée en marquant un aptamère spécifique aux protéines à abondance élevée avec le marqueur fluorescent ou UV et fixant cet aptamère marqué par un marqueur aux protéines à abondance élevée.

5. Procédé selon la revendication 3, dans lequel la protéine à abondance élevée est l'albumine et le peptide spécifique à la protéine à abondance élevée est l'amyloïde αβ40.

6. Procédé selon la revendication 1, dans lequel la protéine est surveillée indépendamment à différentes bandes de longueur d'onde de détection.

7. Procédé selon la revendication 1, dans lequel deux protéines détectables par fluorescence ou UV ou plus peuvent être mélangées dans du sang simultanément et surveillées à des plages de longueur d'onde de détection correspondantes en même temps, dans lequel les protéines détectables par fluorescence ou UV sont un complexe de protéines à abondance élevée, protéines à faible abondance, ou protéines à abondance élevée et protéines à faible abondance, respectivement.


<110> SNU R&DB FOUNDATION
<120> Real-time monitoring of depletion of high-abundance blood proteins or recovery of low-abundance blood proteins by UV spectrometry
<130> HAN14329PCTEP
<140> European Patent application based on PCT/KR2011/005500
   <141> 2011-07-26
<150> KR10-2010-0072142
   <151> 2010-07-26
<160> 2
<170> Kopatent In 1.71
<210> 1
   <211> 21
   <212> DNA
   <213> Artificial Sequence
<220>
   <223> Sense primer for EGFP coding sequence
<400> 1
   gacaagctta tatggtgagc a 21
<210> 2
   <211> 21
   <212> DNA
   <213> Artificial Sequence
<220>
   <223> Anti-sense primer for EGFP coding sequence
<400> 2
   gccgggatca ctctcgagca c 21